# EUROPEAN PATENT APPLICATION

(11) **EP 4 595 972 A1**
(43) Date of publication of application: **06.08.2025**
(21) Application number: 23873250.7
(22) Date of filing: 27.09.2023
(51) Int. Cl.: A61K 38/10, A61P 25/00, A61P 25/28, A23L 33/18

(54) **PEPTIDE FOR TREATING OR PREVENTING 4R TAUOPATHY**

(30) Priority: 30.09.2022 KR 20220125816
(71) Applicant: GemVax & KAEL Co., Ltd., Daejeon 34036 (KR)
(72) Inventor: KIM, Sang Jae, Seoul 06360 (KR)
(74) Representative: Stolmár & Partner Patentanwälte PartG mbB
(86) International application number: PCT/KR2023/015104
(87) International publication number: WO 2024/072143

(57) **Abstract**

An aspect of the present invention relates to a pharmaceutical composition or food composition for prevention, treatment or improvement of 4R tauopathy, which includes one or more selected from a peptide having an amino acid sequence of SEQ ID NO: 1, a peptide having sequence identity of 80% or more to the amino acid sequence, and a fragment thereof as another peptide, as an active ingredient; or a medical use of the peptide for prevention, treatment or improvement of 4R tauopathy.

## Description

### [Technical Field]

The present invention relates to a peptide having efficacy of treating or preventing 4R tauopathies and a composition including the same, and more particularly, to a peptide selected from telomerases effective for treating or preventing tauopathies, and a composition including the same.

### [Background Art]

Nerve cells or neurons are various types of cells formed by differentiation of a neural stem cell. The neural stem cell is differentiated into a variety of cells such as neuron, glia, astrocyte, oligodendrocyte, and the like, to construct a nervous system.

Causing a disorder in nerve function refers to nervous diseases or neurological disorder, which may include stroke, brain tumor, epilepsy, migraine, sleep disorder, Alzheimer's disease and tauopathy, etc. Here, the tauopathy includes progressive supranuclear palsy, corticobasal degeneration, Pick's disease, frontotemporal lobar degeneration with tau (FTLD-tau), argyrophilic grain disease, subacute sclerosing panencephalitis, Christianson syndrome, post-encephalitic parkinsonism, Guadeloupean parkinsonism, spinocerebellar ataxia type II, chronic traumatic encephalopathy (including traumatic brain injury and dementia pugilistica), globular glial tauopathy, ARTAG (aging-related tau astrogliopathy including globular glial tauopathy), PART (primary age-related tauopathy includes tangle-predominant dementia and clinically asymptomatic cases) and the like.

The tauopathy calls collectively neurodegenerative diseases related to aggregation of tau proteins into neurofibrilary tangle (NFT). With regard to the tauopathy, firstly, it occurs a deformation in which phosphate is excessively adhered to the tau protein to cause hyperphosphorylation of tau, which in turns aggregates and forms the NFT. Such a tau NFT is a type of compressed bunch of filaments, and is shown like as a mass of twisted threads existing in brain cells. Such an abnormal tau aggregation is a major feature of neurodegenerative diseases called tauopathy collectively (Brandt R, Hundelt M, Shahani N (2005) Tau alteration and neuronal degeneration in tauopathies: mechanisms and models. Biochimica. et biophysica. acta. 1739: 331-354).

In a health nerve, tau promotes the growth from axon and polarization of neurons, thereby contributing to stabilization of microtubules. In a pathological aspect, if tau is hyperphosphorylated, it is separated from the microtubules and generates insoluble aggregate (Gendron TF, Petrucelli L (2009) The role of tau in neurodegeneration. Mol. Neurodegener. 4: 13).

Over several years, a structural backbone of tau aggregates has been proposed. Further, there have been proposed some evidences demonstrating that insoluble filaments are formed from 10 soluble monomers, and these filaments are combined in a high order structure called neurofibrillary tangles (NFTs).

Depending on isoform of tau protein contributing to the tauopathy, that is, the predominance of 4R tau or 3R tau, tauopathy appears different neurodegenerative diseases (Front. Neurol., 05 November 2020|https://doi.org/10.3389/fneur.2020.599384; Neuron 90, 941-947, June 1, 2016).

According to the above description, in the pathological aspect, tauopathy may be classified into primary tauopathy having tau deposition as a major pathogenesis, and secondary tauopathy wherein additional pathogens (amyloid, trauma and autoimmunity, etc.) participate in the tau deposition. The primary tauopathy may further be divided into 4R tauopathy or 3R tauopathy depending on the predominance of 4R tau or 3R tau. Alzheimer's disease known as the most common tauopathy is a secondary tauopathy expressed due to participation of amyloid deposition in the tau deposition, and in this aspect, is distinguished from the primary tauopathy. In particular, Alzheimer's disease is the secondary tauopathy wherein amyloid beta is regarded as the major cause of the disease, which is consistently demonstrated by genetic and biochemical evidences (Rosler TW et al., Four-repeat tauopathies. Prog Neurobiol. 2019, 180:101644 https://pubmed.ncbi.nlm.nih.gov/31238088/).

Among the primary tauopathies, 4R tauopathy may include progressive supranuclear palsy, corticobasal degeneration, argyrophilic grain disease, globular glial tauopathy and ARTAG (aging-related tau astrogliopathy including globular glial tauopathy), etc. (Olfati N et al., Clinical Spectrum of Tauopathies. Front Neurol. 2022, 13:944806). Specifically, progressive supranuclear palsy is classified as a representative disease expressed in 4R tauopathy.

Although the number of diseases related to the 4R tauopathy is increasing with the progress of population aging, no specific drug disclosed to effectively treat or prevent the diseases has yet to be proposed till now. Therefore, there is now unsatisfied requirements for drug that is efficiently useable for treatment, improvement or prevention of 4R tauopathy.

Meanwhile, it was reported that 16 amino acid peptides PEP1 (SEQ ID NO: 1) selected from reverse transcriptase of human telomerase (hTERT) have anticancer, antiinflammatory and anti-oxidative efficacies, as well as are effective in treatment of Alzheimer's disease (WO2013/167574). Further, it was confirmed that PEP1 has completed toxicity test in a number of clinical trials targeting different diseases, and exhibits stability to side effects.

LMTM (WO2021-001306) is known as a tau aggregation inhibitor and is now under phase III clinical trials for tauopathy. However, specific and concrete efficacies of the above inhibitor to 4R tauopathy have never been mentioned or experimented in the prior art. Further, it was reported that LMTM shows cytotoxicity side effects (GI₅₀ = 6.4±0.3 µM) in the tau aggregation inhibitory section (EC₅₀ = 2.2±0.2 µM) (Yun Kyung Kim et al. Levosimendan inhibits disulfide tau oligomerization ameliorating tau pathology in TauP301L-BiFC mice, Experimental & Molecular Medicine volume 55, pages 612-627 (2023)).

Further, LMTM involves the most common side effects related to gastrointestinal and urinary system, which were reported as the most general cause for stopping administration of high-dose LMTM (Gauthier S et al., Efficacy and safety of tau-aggregation inhibitor therapy in patients with mild or moderate Alzheimer's disease: a randomised, controlled, double-blind, parallel-arm, phase 3 trial. Lancet. 2016, 388(10062):2873-2884).

### [Prior Art Document]

### [Patent Document]

Patent Document 1. WO2013/167574
Patent Document 2. WO2021/001306

### [Non-Patent Document]

1.Brandt R, Hundelt M, Shahani N (2005) Tau alteration and neuronal degeneration in tauopathies: mechanisms and models. Biochimica. et biophysica. acta. 1739: 331-354
2. Gendron TF, Petrucelli L (2009) The role of tau in neurodegeneration. Mol. Neurodegener. 4: 13
3. Front. Neurol., 05 November 2020 https://doi.org/10.3389/fneur.2020.599384
4. Neuron 90, 941-947, June 1, 2016
5. Rosler TW et al., Four-repeat tauopathies. Prog Neurobiol. 2019, 180:101644 | https://pubmed.ncbi.nlm.nih.gov/31238088/
6. Olfati N et al., Clinical Spectrum of Tauopathies. Front Neurol. 2022, 13:944806
7. Yun Kyung Kim et al. Levosimendan inhibits disulfide tau oligomerization ameliorating tau pathology in TauP301L-BiFC mice, Experimental & Molecular Medicine volume 55, pages 612-627 (2023)
8. Gauthier S et al., Efficacy and safety of tau-aggregation inhibitor therapy in patients with mild or moderate Alzheimer's disease: a randomised, controlled, double-blind, parallel-arm, phase 3 trial. Lancet. 2016, 388(10062):2873-2884.

### [Summary of Invention]

### [Problems to be Solved by Invention]

One aspect of the present invention is to provide a peptide effective for treatment or prevention of 4R tauopathy.

Another aspect of the present invention is to provide a pharmaceutical composition for treatment or prevention of 4R tauopathy, which includes the above peptide.

In addition, another aspect of the present invention is to provide a food composition for improvement or prevention of 4R tauopathy, which includes the above peptide.

Further, another aspect of the present invention is to provide a medical use of the above peptide, in order to treat or prevent 4R tauopathy.

Furthermore, another aspect of the present invention is to provide a method for treatment or prevention of 4R tauopathy, which includes administering the above peptide to a subject who requires treatment or prevention of 4R tauopathy.

### [Means for Solving Problems]

According to an aspect of the present invention, there is provided a pharmaceutical composition for prevention or treatment of 4R tauopathy, which includes one or more selected from a peptide having an amino acid sequence of SEQ ID NO: 1, a peptide having sequence identity of 80% or more to the amino acid sequence, and a fragment thereof as another peptide, as an active ingredient.

According to another aspect of the present invention, there is provided a food composition for prevention or improvement of 4R tauopathy, which includes one or more selected from a peptide having an amino acid sequence of SEQ ID NO: 1, a peptide having sequence identity of 80% or more to the amino acid sequence, and a fragment thereof as another peptide, as an active ingredient.

In addition, according to another aspect of the present invention, there is provided one or more selected from a peptide having an amino acid sequence of SEQ ID NO: 1, a peptide having sequence identity of 80% or more to the amino acid sequence, and a fragment thereof as another peptide, which is used for preventing or treating 4R tauopathy.

Further, according to another aspect of the present invention, there is provided a medical use of one or more selected from a peptide having an amino acid sequence of SEQ ID NO: 1, a peptide having sequence identity of 80% or more to the amino acid sequence, and a fragment thereof as another peptide, which is used for preventing or treating 4R tauopathy.

Furthermore, according to another aspect of the present invention, there is provided a method for treatment or prevention of 4R tauopathy, which includes administering one or more selected from a peptide having an amino acid sequence of SEQ ID NO: 1, a peptide having sequence identity of 80% or more to the amino acid sequence, and a fragment thereof as another peptide in a pharmaceutically effective amount, to a subject who needs the prevention or treatment of 4R tauopathy.

### [Advantageous Effects]

One or more selected from a peptide having an amino acid sequence of SEQ ID NO: 1, a peptide having sequence identity of 80% or more to the amino acid sequence, and a fragment thereof as another peptide exhibited significant improvement of exercise performance in a 4R-Tau-BiFC mouse model introduced with P30L mutant known to induce 4R tauopathy, that is, 4R tauopathy mouse model, as compared to the control and reference drug administration group. Further, in the assessment of improvement in cognitive ability, significant improvement was confirmed. In addition, as a result of brain tissue image assay and brain lysate assay in the 4R-Tau-BiFC mouse model, as compared to the control and reference drug administration group, it was confirmed that effects of inhibiting formation of 4R that causes 4R tauopathy, and 4R tau hyperphosphorylation are excellent. Accordingly, one or more selected from a peptide having an amino acid sequence of SEQ ID NO: 1, a peptide having sequence identity of 80% or more to the amino acid sequence, and a fragment thereof as another peptide were confirmed to effectively inhibit 4R tauopathy, therefore, it is expected that the peptide may be effectively used for treatment, improvement or prevention of 4R tauopathy.

### [Brief Description of Drawings]

FIG. 1 is a schematic view illustrating an administering dose and schedule of a drug for animal-based behavior assessment of PEP1 according to Experimental Example 2.
FIG. 2 is a graph showing results of evaluating motor ability improvement efficacy in a 4R TauP301L-BiFC mouse model through a Rota-rod test. Through bidirectional ANOVA using Tukey's multiple-comparisons, the results were analyzed (*p<0.05; **p<0.01; n.s. Insignificant for vehicle).
FIG. 3 is a graph showing results of evaluating cognitive ability improvement efficacy in 4R TauP301L-BiFC mouse model through a new object recognition test. A black bar graph indicates the result value in regard to the learned object, while a red bar graph indicates the result value in regard to a novel object. Through bidirectional ANOVA using Sidak's multiple comparisons test, the results were analyzed (*p<0.05; ***p<0.0001).
FIG. 4 schematically illustrates the administration dose and schedule of the drug for assessing animal-based 4R-tauopathy inhibitory efficacy of PEP1 according to Experimental Example 3.
FIG. 5 is photographs showing (A) Tau-BiFC imaging and (B) AT8 immunofluorescent staining results in the somatosensory cortical region, as well as graphs showing results of quantitative analysis of fluorescent levels.
FIG. 6 is photographs showing (A) Tau-BiFC imaging and (B) AT8 immunofluorescent staining results in the motor cortical region, as well as graphs showing results of quantitative analysis of fluorescent levels.
FIG. 7 is photographs showing (A) Tau-BiFC imaging and (B) AT8 immunofluorescent staining results in the hippocampus region, as well as graphs showing results of quantitative analysis of fluorescent levels.
FIG. 8 is photographs showing (A) Tau-BiFC imaging and (B) AT8 immunofluorescent staining results in the substantia nigra region, as well as graphs showing results of quantitative analysis of fluorescence levels.

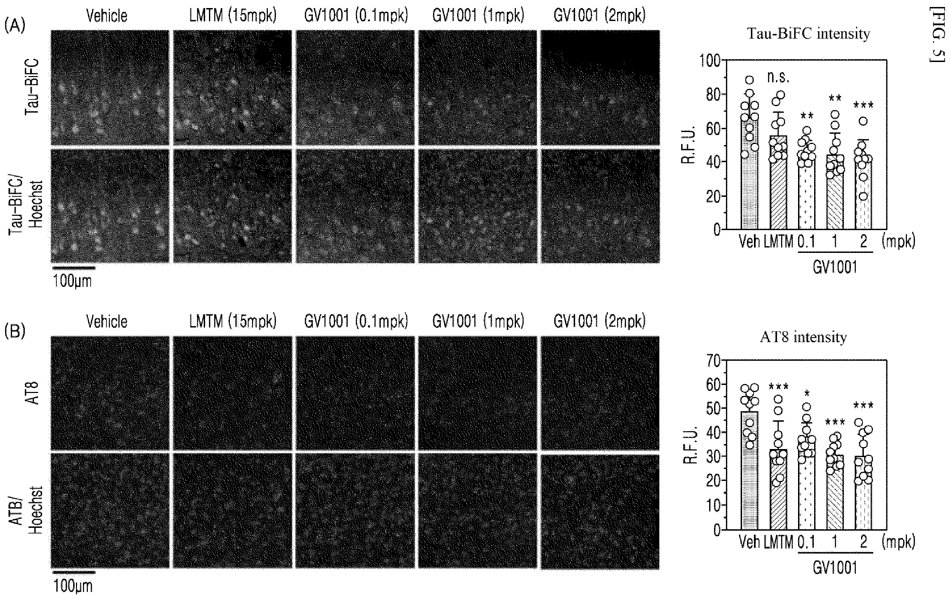

FIGS. 5 to 8 show results of monodirectional ANOVA analysis using Dunnett's multiple-comparisons test, respectively (*p<0.05, **p<0.01, ***p<0.001, n.s.; Insignificant for vehicle).

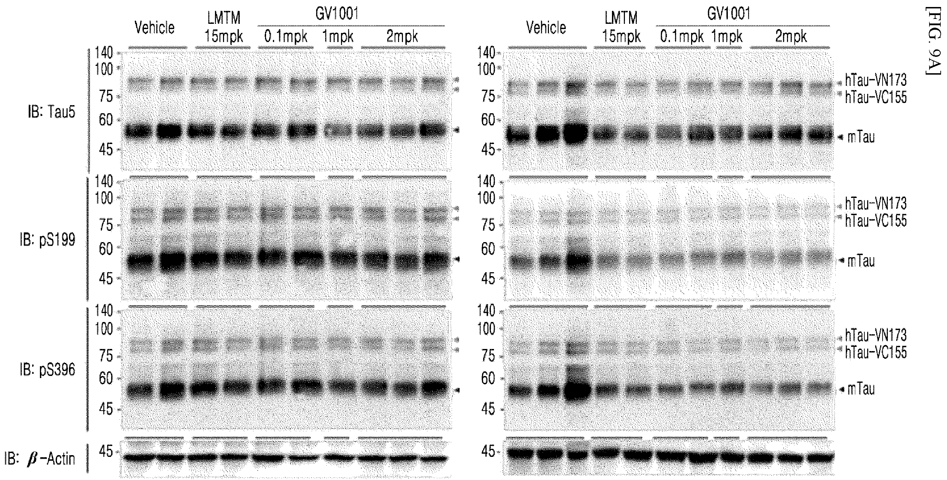

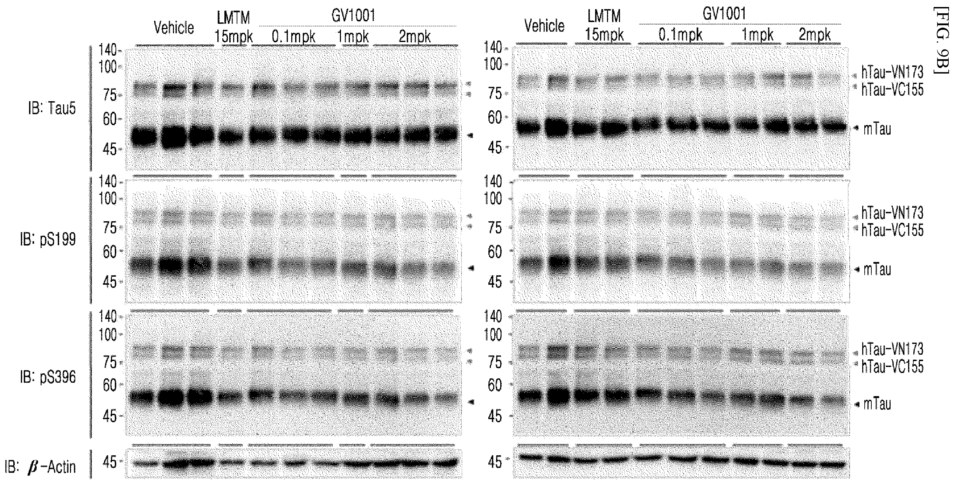

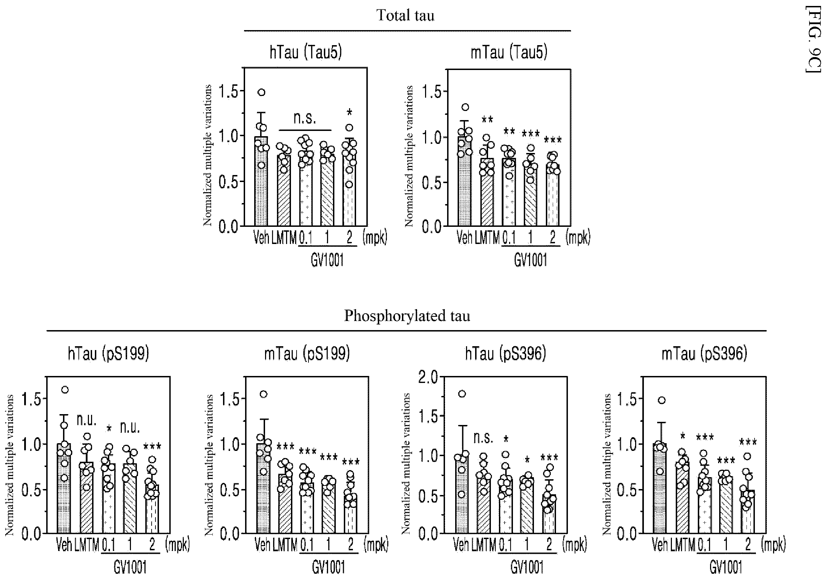

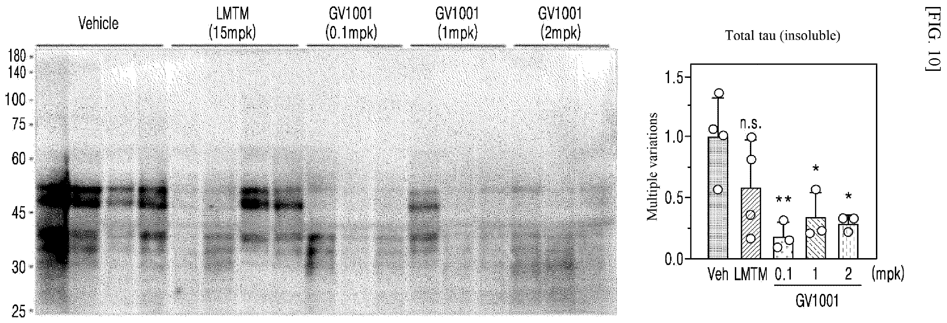

FIGS. 9 and 10 are photographs showing results of immunoblot using total tau (Tau5) and phosphorylated tau (pS199, pS396) antibodies and graphs showing results of quantitative analysis of total tau and phosphorylated tau levels, wherein these results were obtained by: preparing brain lysate of each mouse in the TauP301L-BiFC mouse model including 7 to 12 month-old mice administered with the drug for 5 months; and using soluble fractional samples (FIG. 9A: immunoblot photographs showing somatosensory cortex and motor cortex, respectively; FIG. 9B: immunoblot photographs showing hippocampus and substantia nigra, respectively; and FIG. 9C: a graph showing results of tau quantitative analysis) and insoluble fractional sample (FIG. 10). Through monodirectional ANOVA using Dunnett's multiple-comparisons test, the above results were analyzed (*p<0.05, **p<0.01, ***p<0.001, n.s.; Insignificant for vehicle).

### [Mode for Carrying out Invention]

Hereinafter, the present invention will be described in more detail in order to help understanding of the present invention.

All technical terms used in the present invention are used with the same meanings as generally understood by those skilled in the art to which the present invention pertains, unless otherwise defined. Further, although preferred methods or samples are described in the present specification, others similar or equal to the same are also included in the scope of the present invention. Further, the numeral values described in the present specification are regarded to include the meanings of "about" even though not clarified. The contents of all publications introduced as reference literatures in the present application are incorporated herein by reference in those entireties.

In the present application, the term "4R tauopathy" means neurodegenerative diseases occurring due to the predominance of specifically 4R tau aggregation in isoforms of tau protein among tauopathies, and may include progressive supranuclear palsy, corticobasal degeneration, argyrophilic grain disease, globular glial tauopathy, and ARTAG (aging-related tau astrogliopathy including globular glial tauopathy), etc., but it is not limited thereto.

The inventors have found that PEP1 as a peptide selected from telomerases (hereinafter, also referred to as "GV1001") is effective in inhibiting 4R tauopathy. More particularly, through behavior test and analysis of brain tissues using 4R-Tau-BiFC mouse model introduced with P301L mutant known to induce 4R tauopathy (that is, an animal model with 4R tauopathy) (Acta Neuropathol (2017) 133:665-704), it was confirmed that PEP1 inhibits 4R tau formation and 4R tau hyperphosphorylation as causes of 4R tauopathy (Experimental Example 3), and actually improves motor ability and cognitive ability in 4R tauopathy animal model (Experimental Example 2). Further, as compared to LMTM, a tauopathy drug conventionally known to involve concerns such as cytotoxicity, gastrointestinal and urological side effects, etc., it was confirmed that PEP1 improves cognitive ability and motor ability of a subject with 4R tauopathy while inhibiting 4R tau formation and 4R tau hyperphosphorylation as causes of 4R tauopathy, even at a considerably low dose. Such effects of GV1001, which is known as a very stable peptide without concerns about side effects conventionally known in the art (Hyun-Hee Park et al., Neurobiology of Aging (2014) 35(6):1255-74; Hyun-Hee Park et al., Neurotoxicology (2016) 55:131-141)) on 4R tauopathy could be safely used for effective treatment or prevention of tauopathy as compared to LMTI known as a conventional simple tauopathy drug. Further, it was originally confirmed that GV1001 is effective for specifically 4R tauopathy. As such, the above results show great technical significance of the present invention.

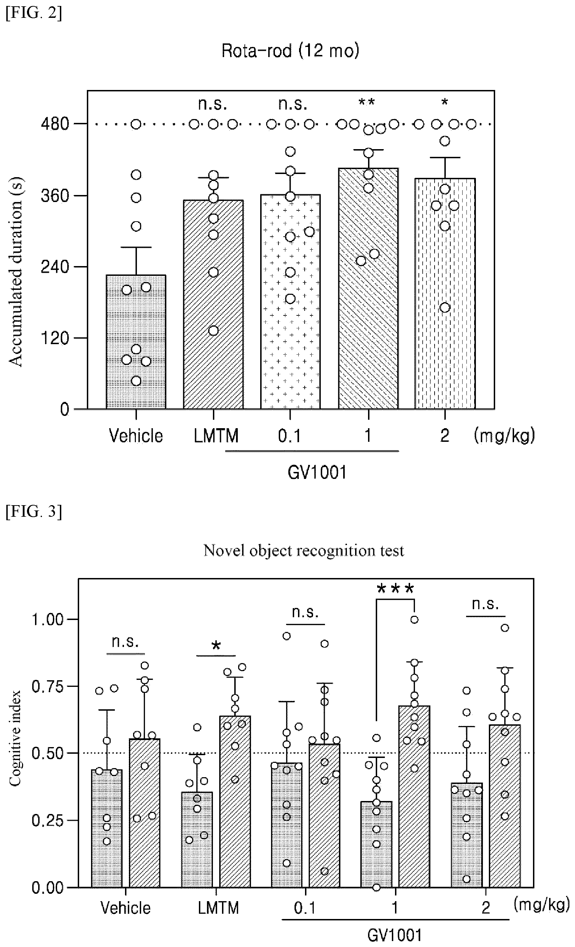

More concretely, according to Experimental Example 2, as a result of evaluating motor ability improvement efficacy through a Rota-rod test (FIG. 2) in a 4R TauP301L-BiFC mouse model including 7- to 12-month-old mice administered with the drug for 5 months, PEP1 1 and 2 mg/kg administration groups exhibited significant improvement in exercise performance, as compared to the vehicle (control) and reference compound LMTM. Further, as a result of evaluating cognitive ability improvement efficacy through novel object recognition test (FIG. 3) in 4R TauP301L-BiFC mouse model, PEP1 administration group showed significant improvement in cognitive ability to the novel object, as compared to the vehicle and reference compound LMTM.

Further, according to Experimental Example 3, (A) Tau-BiFC imaging and (B) AT8 immunofluorescent staining was performed using brain tissues of each mouse in the 4R TauP301L-BiFC mouse model including 7- to 12-month-old mice administered with the drug for 5 months. As a result of quantitative analysis of fluorescent levels of Tau-BiFC in somatosensory cortex, motor cortex, hippocampus (CA1) and substantia nigra regions where 4R-tauopathic pathology due to formation of tau oligomer is predominantly expressed (FIGS. 5 to 8), PEP1 administration group had significant reduction in fluorescent intensity, as compared to the vehicle and reference compound LMTM administration groups, thereby exhibiting tau oligomer formation inhibitory efficacy. Further, when performing immunofluorescent staining using tau hyperphosphorylation antibodies, that is, AT8 (phospho-Tau S202/T205), PEP1 administration group also had significant reduction in AT8 fluorescent intensity as compared to the vehicle and reference compound LMTM administration group, thereby exhibiting 4R tau hyperphosphorylation inhibitory efficacy. Further, after obtaining the brain lysate of each mouse in the TauP301L-BiFC mouse model including 7- to 12-month-old mice administered with the drug for 5 months, immunoblot was conducted using total tau (Tau5) and phosphorylated tau (pS199, pS396) antibodies, along with (i) a soluble fraction sample and (ii) an insoluble fraction sample (FIGS. 9 and 10). As a result of the above analysis, it was confirmed that: as compared to the vehicle and reference compound LMTM administration groups, the PEP1 administration group in the soluble fraction sample had significant reduction in total tau and phosphorylated tau levels; whereas the PEP1 administration group in the insoluble fraction sample showed significant efficacy of inhibiting formation of insoluble tau aggregate.

According to an aspect of the present invention, there is provided a pharmaceutical composition for prevention or treatment of 4R tauopathy, which includes one or more selected from a peptide having an amino acid sequence of SEQ ID NO: 1, a peptide having sequence identity of 80% or more to the amino acid sequence, and a fragment thereof as another peptide as another peptide, as an active ingredient.

According to another aspect of the present invention, there is provided a food composition for prevention or improvement of 4R tauopathy, which includes one or more selected from a peptide having an amino acid sequence of SEQ ID NO: 1, a peptide having sequence identity of 80% or more to the amino acid sequence, and a fragment thereof as another peptide as another peptide, as an active ingredient.

In one embodiment, the fragment is a peptide as a fragment consisting of three or more amino acids.

The 4R tauopathy may be selected from progressive supranuclear palsy, corticobasal degeneration, argyrophilic grain disease, globular glial tauopathy and ARTAG (aging-related tau astrogliopathy including globular glial tauopathy), but it is not limited thereto, and further include any neurodegenerative disease occurring due to the predominance of 4R tau aggregation.

The peptide disclosed in the present specification may include a peptide having sequence identity of 85% or more, 90% or more, 95% or more, 96% or more, 97% or more, 98% or more, or 99% or more. Further, the peptide disclosed in the present specification may include a peptide having SEQ ID NO: 1 or fragments thereof, and peptides with modification of one or more amino acids, 2 or more amino acids, 3 or more amide acids, 4 or more amino acids, 5 or more amino acids, 6 or more amino acids, or 7 or more amino acids.

In one embodiment, the modification of amino acids may belong to a variation to alter physicochemical features of the peptide. For example, a modification of amino acid such as enhancing thermal stability of the peptide, altering substrate specificity, varying optimum pH, etc. may be performed.

As used herein, the "amino acid" may include D-isomer and modified amino acids as well as 22 standard amino acids naturally integrated into peptides. Therefore, in one embodiment, the peptide may be a peptide containing D-amino acid.

In one embodiment, the peptide may include non-standard amino acid modified after translation (i.e., post-translational modification) or the like. The post-translational modification includes phosphorylation, glycosylation, acylation (e.g., including acetylation, myristoylation, palmitoylation), alkylation, carboxylation, hydroxylation, glycation, biotinylation, ubiquitinylation, variation in chemical properties (e.g., beta-removal deimidation, deamidation) and structural change (e.g., formation of bisulfide bridge). Further, the peptide includes a change in amino acid caused by chemical reactions occurring in a process for combination of the peptide with crosslinkers for forming peptide conjugates, for example, a change of amino acid such as variation in an amino group, a carboxyl group or side chains.

The peptide may be a wild-type peptide identified and isolated from natural resources. Meanwhile, the peptide may be an artificial variant in which one or more amino acids have substituted, deleted and/or inserted amino acid sequence, as compared to the peptide of SEQ ID NO: 1. Changes of amino acid in the wild-type peptide as well as the artificial variant may include a conservative amino acid substitution that does not significantly affect folding and/or activity of a protein. Examples of the conservative substitutes may be included in the group of basic amino acids (arginine, lysine and histidine), acidic amino acids (glutamic acid and aspartic acid), polar amino acids (glutamine and asparagine), hydrophobic amino acids (leucine, isoleucine, valine and methionine), aromatic amino acids (phenylalanine, tryptophan and tyrosine), and small amino acids (glycine, alanine, cerine and threonine). General amino acid substitution without altering specific activity is known in the art. Commonly occurring substitution may include Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu and Asp/Gly, and the others opposed thereto. Other examples of the conservative substitution may include those listed in Table 1 below.

**[TABLE 1]**

| Original amino acid | Examples of residual substitute | Preferable residual substitute |
|---|---|---|
| Ala (A) | val; leu; ile | Val |
| Arg (R) | lys; gln; asn | Lys |
| Asn (N) | gln; his; asp, lys; arg | Gln |
| Asp (D) | glu; asn | Glu |
| Cys (C) | ser; ala | Ser |
| Gln (Q) | asn; glu | Asn |
| Glu (E) | asp; gln | Asp |
| Gly (G) | Ala | Ala |
| His (H) | asn; gln; lys; arg | Arg |
| Ile (I) | leu; val; met; ala; phe; norleucine | Leu |
| Leu (L) | norleuicine; ile; val; met; ala; phe | Ile |
| Lys (K) | arg; gln; asn | Arg |
| Met (M) | leu; phe; ile | Leu |
| Phe (F) | leu; val; ile; ala; tyr | Tyr |
| Pro (P) | Ala | Ala |
| Ser (S) | Thr | Thr |
| Thr (T) | Ser | Ser |
| Trp (W) | tyr; phe | Tyr |
| Tyr (Y) | trp; phe; thr; ser | Phe |
| Val (V) | ile; leu; met; phe; ala; norleucine | Leu |

Real modification of biological features of the peptides may be performed by selecting quite different substitutes in terms of their: (a) effects on maintaining a structure of polypeptide backbone in the substitution region, for example, a sheet or helical 3D structure; (b) effects on maintaining charges or hydrophobic properties of molecules in a target site; or (c) effects on maintaining side chains bulky. Natural residues may be classified into the following groups based on general features of side chains:
(1) Hydrophobic property: nor-leucine, met, ala, val, leu, ile;
(2) Neutral hydrophilic property: cys, ser, thr;
(3) Acidic property: asp, glu;
(4) Basic property: asn, gln, his, lys, arg;
(5) Residues that affect orientation of chains: gly, pro; and
(6) Aromatic: trp, tyr, phe.

The non-conservative substitution will be performed by exchanging one member among the above classes into another class. Any cysteine residue independent of maintaining a proper 3D structure of the peptide may be generally substituted with serine to improve oxidative stability of the molecule, and can prevent an abnormal cross-linked bond. Inversely, adding cysteine bond(s) to the peptide may enhance stability thereof.

Another type of amino acid variant of the peptide is one having varied glycosylation pattern of an antibody. The variation indicates deletion of one or more hydrocarbon residues found in the peptide and/or addition of one or more glycosylation sites, which are not present in the peptide.

Glycosylation of peptide means typically N-linked or O-linked one. The "N-linked" one means that a hydrocarbon residue is attached to a side chain of an asparagine residue. Tripeptide sequence asparagine-X-serin and asparagine-X-threonine (wherein X is any amino acid except for proline) is a recognition sequence for enzymic adhesion of a hydrocarbon residue to an asparagine side chain. Therefore, one of these tripeptide sequences exists in polypeptide thus to generate a potential glycosylation site. The O-linked glycosylation means that one of glucose n-acetyl galactosamine, galactose or xylose is attached to hydroxyamino acid, most commonly, serine or threonine, however, 5-hydroxyproline or 5-hydroxylysine may also be used.

Addition of glycosylation site to the peptide may be easily performed by varying the amino acid sequence so as to contain one or more of the above-mentioned tripeptide sequences (in the case of N-linked glycosylation site). Such variation may also be performed by adding one or more serin or threonine residues to the sequence of an original antibody or substituting the original one with these residues (in the case of O-linked glycosylation site).

Further, as an active ingredient, the peptide having an amino acid sequence of SEQ ID NO: 1, a peptide having sequence identity of 80% or more to the amino acid sequence, and a fragment thereof as another peptide, have advantages of low toxicity in the cells, and high stability *in vivo.*

The peptide having an amino acid sequence of SEQ ID NO: 1 is shown in Table 2 below. The "name" in Table 2 is given to distinguish different peptides. In Table 2 below, the peptide of SEQ ID NO: 2 indicates whole peptide of human telomerase. In one embodiment, the peptide having the amino acid sequence of SEQ ID NO: 1 includes a peptide of SEQ ID NO: 2. In one embodiment, the peptide having the amino acid sequence of SEQ ID NO: 1 includes the peptide consisting of the amino acid sequence of SEQ ID NO: 1. The peptide having the amino acid sequence of SEQ ID NO: 1, a peptide having sequence identity of 80% or more to the amino acid sequence, and a fragment thereof as another peptide, may include any "synthetic peptide" synthesized after selecting a peptide at the corresponding position among peptides included in a telomerase. SEQ ID NO: 2 indicates the amino acid sequence of whole telomerase.

**[TABLE 2]**

| **SEQ ID NO.** | **Name** | **Position in telomerase** | **Sequence** | **Length** |
|---|---|---|---|---|
| 1 | pep1 | [611-626 ] | EARPALLTSRLRFIPK | 16 aa |
| 2 | | [1-1132] | | 1132 aa |

In one embodiment, the pharmaceutical composition may include a peptide including an amino acid sequence of SEQ ID NO: 1, a peptide having sequence identity of 80% or more to the amino acid sequence or a fragment thereof as another peptide at a concentration of 0.01 to 100 mg/mL, and preferably 0.1 to 10 mg/mL. However, if there is a difference in effects depending on dose for each subject to be treated, the concentration may be appropriately adjusted. When the peptide is included in the above range or less, it may suitably exhibit effects intended in the present invention, as well as satisfy both stability and safety of the composition. Furthermore, the peptide included in the above range may also desirable in an aspect of effects to costs.

The pharmaceutical composition may be applicable to all animals including human, dog, chicken, pig, cow, ram, guinea pig or monkey.

The pharmaceutical composition may be administrated through any route such as oral, intrarectal, transdermal, intravenous, intramuscular, intraperitoneal, intramedullary, intradural, intradermal or subcutaneous injection, etc., preferably intraperitoneal, intradermal, vesicular or subcutaneous injection, and more preferably subcutaneous injection.

Formulations for oral administration may include tablets, pills, soft or hard capsules, granules, powder, liquid or emulsion, but they are not limited thereto. Formulations for parenteral administration may include injection, dripping agent, lotion, ointment, gel, cream, suspension, emulsion, suppository, patch or aerosol, but they are not limited thereto.

If necessary, the pharmaceutical composition may include additives such as diluent, excipient, glydent, binder, disintegrant, buffer, dispersant, surfactant, colorant, flavoring agent or sweetener, etc. The pharmaceutical composition may be manufactured by any conventional pharmaceutical method known in the art.

Further, the pharmaceutical composition may be administered alone or in combination with one or more additional therapeutics. In one embodiment, the one or more additional therapeutics are active ingredients known as a conventional therapeutic agent for neurodegenerative diseases known in the art. The "administration in combination" means that the pharmaceutical composition according to an aspect of the invention and one or more additional therapeutics are administered simultaneously or in any order at different times with a time interval. Therefore, individual components may be administered separately or at sufficiently contiguous times so as to offer desired treatment effects.

A dosage of the pharmaceutical composition may be different according to age, gender, weight, pathogenic conditions and severity thereof of a subject to be administered, administration route, or decision of a prescriber. Determination of the dosage based on the above factors is within the level of those skilled in the art. For example, daily administration dose may be 0.0001 to 100 mg/kg/day, preferably 0.001 to 10 mg/kg/day, and more preferably 0.001 to 1 mg/kg/day, however, may be appropriately adjusted if there is a difference in effects depending on the dose.

Determination of the administration dose and interval of an active ingredient in the pharmaceutical composition is within the level of those skilled in the art, for example, a unit administration dose for human may range from 0.1 to 100 mg/kg based on a 60 kg weighed adult, more particularly about 0.1 to 50 mg/kg, 0.1 to 30 mg/kg, or 0.5 to 10 mg/kg may be administered, and the administration interval may range from 1 to 4 times per day or 1 to 4 times per week, but it is not limited thereto.

According to another aspect of the present invention, there is provided one or more selected from a peptide having an amino acid sequence of SEQ ID NO: 1, a peptide having sequence identity of 80% or more to the amino acid sequence, and a fragment thereof as another peptide, which is used for preventing or treating 4R tauopathy.

In addition, according to another aspect of the present invention, there is provided a medical use of one or more selected from a peptide having an amino acid sequence of SEQ ID NO: 1, a peptide having sequence identity of 80% or more to the amino acid sequence, and a fragment thereof as another peptide, which is used for preventing or treating 4R tauopathy.

Further, according to another aspect of the present invention, there is provided a method for treatment or prevention of 4R tauopathy, which includes administering one or more selected from a peptide having an amino acid sequence of SEQ ID NO: 1, a peptide having sequence identity of 80% or more to the amino acid sequence, and a fragment thereof as another peptide in a pharmaceutically effective amount, to a subject who needs the prevention or treatment of 4R tauopathy.

In regard to details of the peptide used for preventing or treating 4R tauopathy, the medical use thereof for preventing or treating 4R tauopathy, and the method for treatment or prevention of 4R tauopathy according to the above-listed aspects, the detailed description for the pharmaceutical composition according to the aspect of the present invention, which were described above, will be directly applied.

Further, in regard to details of the peptide used for preventing or treating 4R tauopathy, the medical use thereof for preventing or treating 4R tauopathy, and the administering method used for the method of treating or preventing 4R tauopathy according to the above-listed aspects, the detailed description for the administering dose of the pharmaceutical composition in an amount effective in treatment or prevention of the individual or patient, administering interval, administering times, etc., which were described above, will be directly applied.

Hereinafter, the configuration and effects of the present invention will be more concretely described by way of examples and experimental examples. However, the following examples and experimental examples are provided only for illustrative purposes in order to help understanding of the present invention, and the scope and range of the present invention are not limited thereto.

### Experimental Example 1: Synthesis of peptide PEP-1

A peptide consisting of 16 amino acids having a structure of Formula 1 below, which includes SEQ ID NO: 1 (PEP-1) and is selected from human telomerases, was synthesized.

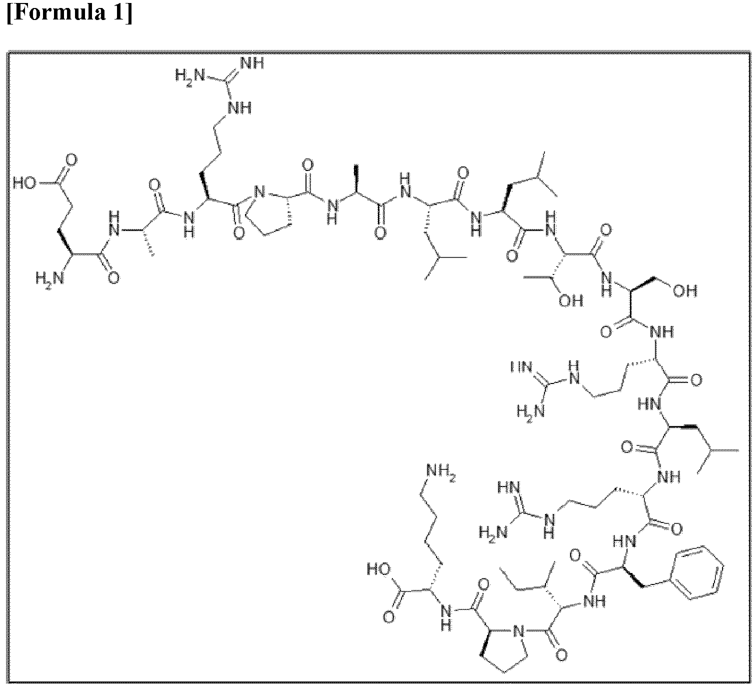

The peptide PEP1 of SEQ ID NO: 1 was prepared by a solid peptide synthesis method known in the art. Specifically, peptides were synthesized by coupling amino acids one by one from C-terminal through Fmoc solid phase peptide synthesis (SPPS) using ASP48S (Peptron, Inc., Daejeon, Korea). As described below, the one in which the first amino acid at C-terminal in the peptides is attached to a resin, was used. For example, the following compounds were used.
NH₂-Lys(Boc)-2-chloro-Trityl Resin
NH₂-Ala-2-chloro-Trityl Resin
NH₂-Arg(Pbf)-2-chloro-Trityl Resin

Every amino acid raw material used in peptide synthesis is characterized in that N-terminal is protected by Fmoc, and the residue is protected by Trt, Boc, t-Bu (t-butylester), Pbf (2,2,4,6,7-pentamethyl dihydro-benzofuran-5-sulfonyl), etc. which are removed in acid. For example, the following compounds were used:
Fmoc-Ala-OH, Fmoc-Arg(Pbf)-OH, Fmoc-Glu(OtBu)-OH, Fmoc-Pro-OH, Fmoc-Leu-OH, Fmoc-Ile-OH, Fmoc-Phe-OH, Fmoc-Ser(tBu)-OH, Fmoc-Thr(tBu)-OH, Fmoc-Lys(Boc)-OH, Fmoc-Gln(Trt)-OH, Fmoc-Trp(Boc)-OH, Fmoc-Met-OH, Fmoc-Asn(Trt)-OH, Fmoc-Tyr(tBu)-OH, Fmoc-Ahx-OH, Trt-Mercaptoacetic acid.

As a coupling reagent, HBTU[2-(1H-Benzotriazole-1-yl)-1,1,3,3-tetamethylaminium hexafluorophosphate] / HOBt [N-Hydroxxybenzotriazole] / NMM [4-Methylmorpholine] were used. Fmoc removal was conducted using 20% piperidine in DMF. The synthesized peptide was separated from the resin while a protective group in the residue was removed using a cleavage cocktail [TFA (trifluoroacetic acid) / TIS (triisopropylsilane) / EDT (ethanedithiol) / H₂O=92.5/2.5/2.5/2.5].

An amino acid protective group-bound starting amino acid is bound to a solid phase support. Under this state, a process of reacting the corresponding amino acids separately, washing the same with a solvent and deprotecting the product was repeated thus to synthesize a peptide. The synthesized peptide was cut off from the resin and purified by HPLC, followed by checking whether the peptide was synthesized or not through MS and then freeze-drying.

A specific synthesis process of PEP1 will be described as follows.

### 1) Coupling

Amino acid (8 equiv.) protected with NH₂-Lys(Boc)-2-chlorotrityl resin and a coupling reagent, that is, HBTU (8 equiv.) / HOBt (8 equiv.) / NMM (16 equiv.) were dissolved in DMF and then added, followed by reacting at room temperature for 2 hours. The product was washed with DMF, MeOH and DMF in this order.

### 2) Fmoc deprotection

After adding 20% piperidine in DMF, the mixture was reacted twice at room temperature for 5 minutes, followed by washing with DMF, MeOH and DMF in this order.
3) A peptide basic backbone was prepared by repeating the reactions of the above 1) and 2).
4) Cleavage: After the synthesis was completed, a cleavage cocktail was added to the peptide resin to separate the peptide from the resin.
5) After adding cooling diethyl ether to the obtained mixture, the mixture was centrifuged to precipitate the obtained peptide.
6) After purifying with Pre-HPLC, a molecular weight was determined through LC/MS, followed by freezing to prepare powder.

### Experimental Example 2: Animal based behavior assessment of GV1001

### Experimental Example 2-1: Summary of experimental progress

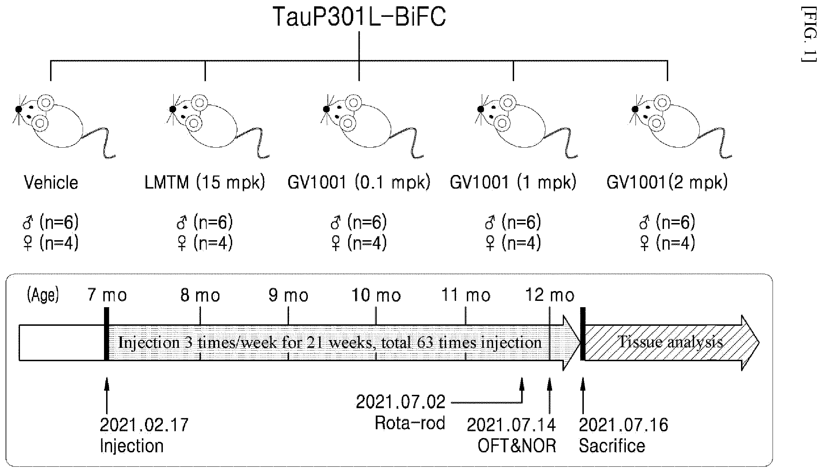

Experimental animals and administration of drugs thereto are shown in Table 3 below and FIG. 1.

**[TABLE 3]**

| | |
|---|---|
| Animal | TauP301L-BiFC mice |
| Age | 7 months old (First injection) |
| Test compound | GV1001 (0.1, 1, 2 mg/kg dissolved in saline)-subcutaneous injection |
| Reference compound | LMTM (15 mg/kg dissolved in saline) - oral injection |
| | Male: 130 uL (average body weight: 30.8 g) |
| | Female: 100 uL (average body weight: 23.4 g) |
| Vehicle | Saline - subcutaneous injection |
| Injection volume for subcutaneous injection | 5 mg/kg |
| | Male: 130 uL (average body weight: 30.8 g) |
| | Female: 100 uL (average body weight: 23.4 g) |
| Injection period | 3 times/week for 21 weeks, total 63 injections |
| Behavior experiments | 1) Rota-rod test on 11.9 months |
| | 2) Novel object recognition test on 12.4 months |

### Experimental Example 2-2: Experimental method

### 1) Rota-rod test method

(1) After carrying out adaptation training for 3 days, a behavior experiment was performed on day 4.
(2) 1 hour before the experiment, a time for adaptation of experimental mice in a space where the behavior experiment is conducted, should be ensured.
(3) During the period for adaptation training, each mouse is forced to walk on a treadmill for 360 seconds at a speed of 5 rpm, 15 rpm, 20/25 rpm. Here, an experimental mouse losing a balance and fallen to the floor was forced to walk again.
(4) After the adaptation training, the behavior experiment was conducted by gradually increasing the speed from 5 rpm to 35 rpm for 480 seconds. When the treadmill worked, a time (seconds) taken until the experimental mouse lost the balance and fallen to the floor was measured.

### 2) Test method for recognition of novel object

(1) On day 1, adaptation training to a new open space was conducted. After an adaptation time for two similar objects added to the corresponding open space on day 2, an experiment to evaluate a cognitive ability to the newly replaced object on day 3 was conducted.
(2) 1 hour before the experiment, a time for adaptation of experimental mice in a space wherein the behavior experiment is conducted, should be ensured.
(3) On day 1, after exposing the experimental mouse to an open space in a size of 40 x 40 cm, the mouse was subjected to behavior assessment for 10 minutes.
(4) On day 2, two similar objects were placed in the same open space, followed by enabling the mouse to learn the objects for 10 minutes.
(5) On day 3, one among the two objects learned in the same open space was replaced with a new object. At this time, a cognitive ability to the replaced object was evaluated.

### Experimental Example 2-3: Results of behavior experiment

### 1) Experiment for assessment of motor ability improvement through Rota-rod test

Results of evaluating motor ability improvement efficacy through a Rota-rod test in a 4R TauP301L-BiFC mouse model were shown in Table 4 below and FIG. 2. Using a TauP301L-BiFC mouse model including 7- to 12-month-old mice administered with the drug for 5 months, the experiment was conducted. The results were analyzed through bidirectional ANOVA using Tukey's multiple-comparisons (*p<0.05; **p<0.01; n.s.: Insignificant for vehicle).

**[TABLE 4]**

| Rota-rod test on 11.9 months | | | | | | | |
|---|---|---|---|---|---|---|---|
| Analysis factor | LMTM | GV1001 | | | | | |
| | vehicle | vs. Vehicle | | | vs. LMTM | | |
| | 15 (mg/kg) | 0.1 (mg/kg) | 1 (mg/kg) | 2 (mg/kg) | 0.1 (mg/kg) | 1 (mg/kg) | 2 (mg/kg) |
| Duration | 57% | 61% | 81%** | 73%* | 3% | 15% | 10% |

According to Table 4 above and FIG. 2, the results of exercise performance in the 4R TauP301L-BiFC mouse model are as follows.
- A transformed mouse model including 11.6- to 12.1-month-old mice administered with the drug 59 times, was subj ected to assessment of exercise performance. As an experimental result of exercise performance in a Rota-rod treadmill, the drug group showed relatively higher exercise performance compared to the vehicle.
- In the case of reference compound LMTM administration group (15 mg/kg), exercise performance tended to improve, however, did not show a significant difference.
- As compared to the vehicle, GV1001 1 and 2 mg/kg administration groups showed significant improvement in exercise performance. In the case of GV1001 0.1 mg/kg administration group, improved exercise performance was observed. However, as compared to the vehicle, statistically significant difference was not confirmed.

### 2) Experiment for assessment of cognitive ability improvement through novel object recognition test

Results of evaluating the cognitive ability improvement efficacy through a novel object recognition test in the 4R TauP301L-BiFC mouse model were shown in FIG. 3. The experiment was conducted using the TauP301L-BiFC mouse model including 7- to 12-month-old mice administered with the drug for 5 months. Black bar graphs indicate the result value for the learned object while oblique bar graphs indicate the result value for the new object. The obtained results were analyzed through bidirectional ANOVA using Sidak's multiple comparisons test (*p<0.05; ***p<0.0001).

According to FIG. 3, the results of comparing the cognitive abilities in the 4R TauP301L-BiFC mouse model are as follows.
- A transformation mouse model including 12.1- to 12.4-month-old mice administered with the drug 62 times, was subjected to evaluation of cognitive ability. From behavior exhibited after replacing the learned object by a new object in the transformed mouse model, the cognitive ability was evaluated. As compared to the vehicle, the result of behavior experiments for the drug administration group showed that a searching time of the new object is longer than that of the learned object.
- In the case of reference compound LMTM administration group (15 mg/kg), it was confirmed that the cognitive ability for the new object is significantly improved.
- GV1001 1 mg/kg administration group showed significant improvement in cognitive ability to the drug. Further, 0.1 mg/kg administration group did not show a great difference compared to the vehicle. Further, 2 mg/kg administration group tended to improve the cognitive ability, but, statistically significant difference was not confirmed.

### Experimental Example 3: Assessment of animal-based 4R-tauopathy inhibitory efficacy by GV1001

### Experimental Example 3-1: Summary of experimental progress

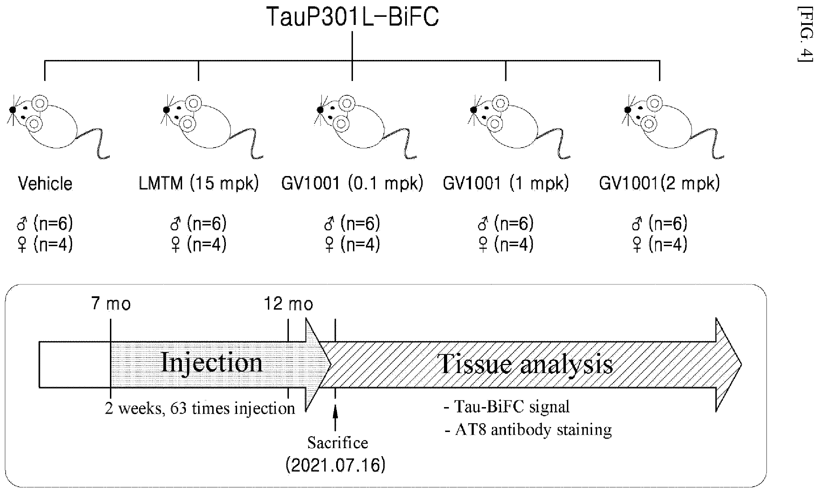

The experimental animals, and drug administration thereto were performed as shown in Table 5 below and FIG. 4.

**[TABLE 5]**

| | |
|---|---|
| Animal | TauP301L-BiFC mice |
| Age | 7 months old (First injection) |
| Test compound | GV1001 (0.1, 1, 2 mg/kg dissolved in saline)-subcutaneous injection |
| Reference compound | LMTM (15 mg/kg dissolved in saline) - oral injection |
| | Male: 130 uL (average body weight: 30.8 g) |
| | Female: 100 uL (average body weight: 23.4 g) |
| Vehicle | Saline - subcutaneous injection |
| Injection volume for subcutaneous injection | 5 mg/kg |
| | Male: 130 uL (average body weight: 30.8 g) |
| | Female: 100 uL (average body weight: 23.4 g) |
| Injection period | 3 times/week for 21 weeks, total 63 injections |
| Brain tissue analysis | 1) Tau-BiFC signal analysis |
| | 2) AT8 (PHF-tau; pSer202/Thr205) |
| Brain lysate analysis | Tau immunoblot |
| | 1) Tay-5 (total tau) antibody |
| | 2) pS199 & pS396 (phospho-tau) antibody |

Experimental reagents used herein are as follows:

### 1) Brain tissue analysis

**[TABLE 6]**

| **Reagent** | **Cat. #** | **Supplier** |
|---|---|---|
| RIPA Buffer | R0278 | Sigma-Aldrich |
| 10% SDS | ML 009-01 | Welgene |
| Sudan Black B | 1999664 | Sigma-Aldrich |
| IgG (H+L) Cross-Adsorbed Goat anti-Mouse. | A21050 | Invitrogen |
| Alexa Fluor^{™} 633 | | |
| Trypsin-EDTA | 25-053-CI | Cellgro |
| DMSO | D2650 | Sigma-Aldrich |
| Phospho-Tau(Ser202. Thr205) | MN1020 | Invitrogen |
| Monoclonal Antibody (ATB) | | |
| Hoechst 33342 | H3570 | Invitrogen |
| DPBS | LB 001-02 | Welgene |
| Paraformaldehyde, powder, 95% | 158127 | Sigma-Aldrich |
| Protease inhibitor cocktail | P8340 | Sigma-Aldrich |
| Phosphatase inhibitor cocktail | P0044 | Sigma-Aldrich |
| Microscope Cover glass | 01012424 | Sigma-Aldrich |
| 2,2,2-Tribromoethanol | T48402 | Sigma-Aldrich |
| Untra-tine II insulin Syringe | 328821 | Becton, Dickinson and Company |
| Superfrost Plus Microscope Slides | 12-550-15 | Fisher Scientific |
| Leica 818 | 14035838926 | Leica |
| Surgipath FSC 22 Clear | 3801480 | Leica |

### 2) Immunoblot of brain lysate

**[TABLE 7]**

| **Reagent** | **Cat. #** | **Supplier** |
|---|---|---|
| RIPA Buffer | R0278 | Sigma-Aldrich |
| 10% SDS | ML 009-01 | Welgene |
| Protease inhibitor cocktail | P8340 | Sigma-Aldrich |
| Phosphatase inhibitor cocktail | P0044 | Sigma-Aldrich |
| DNase 1 | 10104159001 | Sigma-Aldrich |
| Sucrose | 84097 | Sigma-Aldrich |
| Bovine serum albumin. fraction V. heat shock isolation | 9048-46-8 | Cellconic |
| 10X Tris/Glycine/SDS Buffer | 1610772 | Bio-rad |
| 10X Tris/Glycine Buffer | 1610771 | Bio-rad |
| Immunoblot PVDF Membranes | 1620177 | Bio-rad |
| 10X TBS | 1706435 | Bio-rad |
| Tween 20 | P9416 | Sigma-Aldrich |
| Anti-Tau antibody [Tau-5] | ab80579 | Abcam |
| Anti-Tau (Phospho S396) | ab109390 | Abcam |
| Anti-Tau (Phospho S199) | ab81268 | Abcam |
| Anti-Beta-Actin antibody | A1978 | Sigma-Aldrich |
| Goat pAb to MS igG (HRP) | ab6789 | Abcam |
| Goat pAb to Rb IgG (HRP) | ab6721 | Abcam |

### Experimental Example 3-2: Experimental method

### A. Experimental protocol for image analysis of TauP301L-BiFC mouse-based brain tissues

1) Experimental method for brain tissue sampling
   (1) On day 1, after completing 63 times administration schedule, the mice of TauP301L-BiFC mouse model were anesthetized, followed by preparing heart laparotomy.
   (2) Saline was introduced into the heart of each mouse in the TauP301L-BiFC mouse model, followed by removing vivo blood using a perfusion pump machine.
   (3) The brain of the mouse was extracted and weighed.
   (4) The brain to be used for tissue staining experiment was immersed in 4% FPA solution and fixed for 24 hours.
   (5) On day 2, after completing fixation, the brain tissues were immersed in 20% sucrose solution to remove water for 24 hours.
   (6) On day 3, the brain tissues were immersed in 30% sucrose solution to remove water for 48 hours.
   (7) On day 5, the brain tissues free of water were taken and extra moisture was removed. Then, the brain tissues and a frozen tissue embedding agent (OCT compound) were placed in a prepared mold, followed by freezing the brain tissues as fast as possible while protecting the tissues. The frozen brain tissue-containing mold was stored in a defreezer at -80°C. Thereafter, using a cryostat for frozen fragment, the tissues were cut into a unit thickness of 30 µm and stored in 0.05% sodium azide solution.

### 2) Experimental method for AT8 immunofluorescent staining of brain tissues

(1) On day 1, a brain tissue sample having a thickness of 30 µm under cryosection was selected for each brain region, immersed in PBS solution, and then washed three times for 10 minutes.
(3) The sample was fixed in 3.7% formaldehyde for 5 minutes.
(4) The sample was washed in PBS solution three times for 10 minutes.
(5) The brain tissue sample was subjected to permeabilization in 0.3% PBS-T solution.
(6) The sample was immersed in 5% BSA in PBS solution for 1 hour for blocking.
(7) The brain tissues were input into 3% BSA in which primary antibody (AT8) was diluted at a ratio of 1:200, 0.1% Tween-20 in PBS solution, followed by overnight (O/N) binding.
(8) On day 2, the brain tissues were immersed in PBS solution, and washed three times for 10 minutes.
(9) The brain tissues were input into 3% BSA in which secondary antibody was diluted at a ratio of 1:500, 0.1% Tween-20 in PBS solution, followed by binding at room temperature for 1 hour.
(10) The brain tissues were immersed in PBS solution, and washed once for 10 minutes.
(11) The brain tissues were immersed in Hoechst (stock conc., 1mg/mL) diluted at a ratio of 1:2000 in PBS solution, followed by nuclei staining.
(12) The brain tissues were immersed in PBS solution, and washed three times for 10 minutes.
(13) Brain tissue fluorescent imaging was performed using a slide scanner.

### 3) Experimental method for lipid staining of brain tissue for Tau-BiFC imaging

(1) On day 1, a brain tissue sample having a thickness of 30 µm under cryosection was selected for each brain region, immersed in PBS solution, and then washed three times.
(2) A solution of 0.05% Sudan Black B in 70% EtOH was stirred overnight (O/N), and then, particles were filtered through 0.22 µm filter.
(3) The selected brain tissue sample was immersed in distilled water (D.W.), and washed three times for 5 minutes.
(4) The brain tissues were immersed in a prepared 0.05% Sudan Black B solution, followed by staining for 10 minutes.
(5) The brain tissues stained in Sudan black B were immersed in 0.1% PBS-T solution, and washed at an interval of 30 seconds to control a degree of staining.
(6) The tissues were washed in D.W. three times for 5 minutes.
(7) In Hoechst solution at a concentration of 0.2 µg/mL in distilled water, the brain tissues were immersed to conduct nuclei staining for 20 minutes.
(8) The tissues were washed in D.W. three times for 5 minutes.
(9) Tau-BiFC imaging was performed using a slide scanner.

### 4) Fluorescent imaging analysis method of brain tissues

(1) In order to obtain fluorescent images of the stained brain tissues, a brain tissue slide was scanned using Zeiss Axio Scan (Zeiss, Oberkochen, Germany).
   - BiFC fluorescence: λex=460-490 nm, λem=500-550 nm
   - AT8 fluorescence: λex=620-640 nm, λem=650-700 nm
   - Hoechst fluorescence: λex=330-375 nm, λem=430-470 nm
(3) For brain tissues per site obtained through imaging, fluorescent value was calculated through Image J software (NIH).

### B. Experimental protocol for analysis of TauP301L-BiFC mouse-based brain lysate

### 1) Brain lysate (soluble fraction) sampling experimental method

(1) After completing administration, the mice of TauP301L-BiFC mouse model were anesthetized, followed by preparing heart laparotomy.
(2) Saline was introduced to the heart of each mouse in the TauP301L-BiFC mouse model, followed by removing vivo blood using a perfusion pump machine.
(3) After removing the blood, the brain was extracted and weighed.
(4) 1 mL RIPA buffer (w/protease/phosphatase inhibitor cocktail) was added to the extracted brain, followed by homogenization.
(5) After moving the homogenized brain lysate to an ep-tube, it was subjected to incubation in 4°C orbital shaker for 2 hours.
(6) After centrifugation at 4°C with a speed of 13,000 rpm for 20 minutes, the supernatant (soluble fraction) was collected in a new ep-tube and stored at -80°C
(7) In order to prepare an immunoblot sample, the concentration of a soluble fraction sample was quantified by Bradford protein quantification method.
(8) To each sample, RIPA buffer and 4X SDS Laemmli sample buffer (w/ 2.5% β-mercaptoethanol for reducing condition) were added to reach a final concentration of 2 mg/mL.
(9) The sample was boiled at 97°C for 5 minutes.

### 2) Brain lysate (insoluble fraction) sampling experimental method

(1) Pellets remaining after moving the supernatant (soluble fraction) were dispersed in 1 mL of 1M sucrose, DNase I (conc. 1 mg/mL) in RIPA buffer.
(2) After centrifugation at 4°C with a speed of 13,000 rpm for 20 minutes, the supernatant was removed.
(3) After resuspension of pellets in 2% SDS solution (1 mL per gram of tissue), the mixture was subjected to incubation at room temperature for 1 hour.
(4) After centrifugation at room temperature with a speed of 13,000 rpm for 1 minute, the supernatant (insoluble fraction) was collected and moved to a new ep-tube, and the same amount of 2X SDS Laemmli sample buffer (w/ 2.5% β-mercaptoethanol for reducing condition) was added thereto.
(5) The sample was boiled at 97°C for 5 minutes.

### 3) Tau immunoblots experimental method

(1) On day 1: 20 µg of brain lysate sample (Soluble/Insoluble fractions) was loaded to 10% SDS-PAGE gel.
(2) Electrophoresis was performed at 80 V for 30 minutes, and after changing the voltage to 90 V, electrophoresis was continued for 1 hour.
   - SDS-PAGE running buffer: 1X Tris/Glycine/SDS buffer in tertiary distilled water
(3) PVDF membrane cut in a size of a transfer tray was immersed in 100% methanol for 1 minute, in autoclaved tertiary distilled water for 3 minutes, and then, in a cold 1X Transfer buffer for 10 minutes.
   - Transfer buffer: 1X Tris/Glycine buffer combined with 20% methanol in tertiary distilled water (1X Tris/Glycine buffer w/ 20% Methanol in 3'D.W.)
(4) Wet-transfer was performed at 100 V for 1 hour 20 minutes.
(5) The membrane was immersed in 5% BSA in TBS-T (w/ 0.1% Tween-20) solution to conduct blocking at room temperature for 1 hour.
(6) The membrane was immersed in 2.5% BSA containing primary antibody in TBS-T (w/ 0.1% Tween-20) solution, followed by binding in an orbital shaker at 4°C overnight (O/N).
   - Anti-Tau (Tau5) antibody (ab80579, 1:5000)
   - Anti-Tau (phospho S199) antibody (ab109390, 1:5000)
   - Anti-Tau (phospho S396) antibody (ab81268, 1:5000)
(7) On day 2: the membrane was immersed in TBS-T (w/ 0.1% Tween-20) solution, and washed three times for 10 minutes.
(8) The membrane was immersed in 2.5% BSA containing secondary antibody in TBS-T (w/ 0.1% Tween-20) solution, followed by binding at room temperature for 1 hour.
   - Goat Anti-Mouse IgG H&L (HRP) secondary antibody (ab6789, 1:10000)
   - Goat Anti-Rabbit IgG H&L (HRP) secondary antibody (ab6721, 1:10000)
(9) The membrane was immersed in TBS-T (w/ 0.1% Tween-20) solution, and washed three times for 10 minutes.
(10) Using ECL solution as a HRP substrate, a band of the membrane was detected through ChemiDoc.
(11) Quantification was performed to measure an intensity of the detected band through Image J software (NIH).

### Experimental Example 3-3: Results of evaluation for animal based 4R-tauopathy inhibitory efficacy of GV1001

A. Results of evaluation for tauopathy improvement efficacy through Tau-BiFC imaging and AT8 immunofluorescent staining
   (A) Tau-BiFC imaging and (B) AT8 immunofluorescent staining were performed using brain tissues of each mouse in the TauP301L-BiFC mouse model including 7- to 12-month-old mice administered with the drug for 5 months.

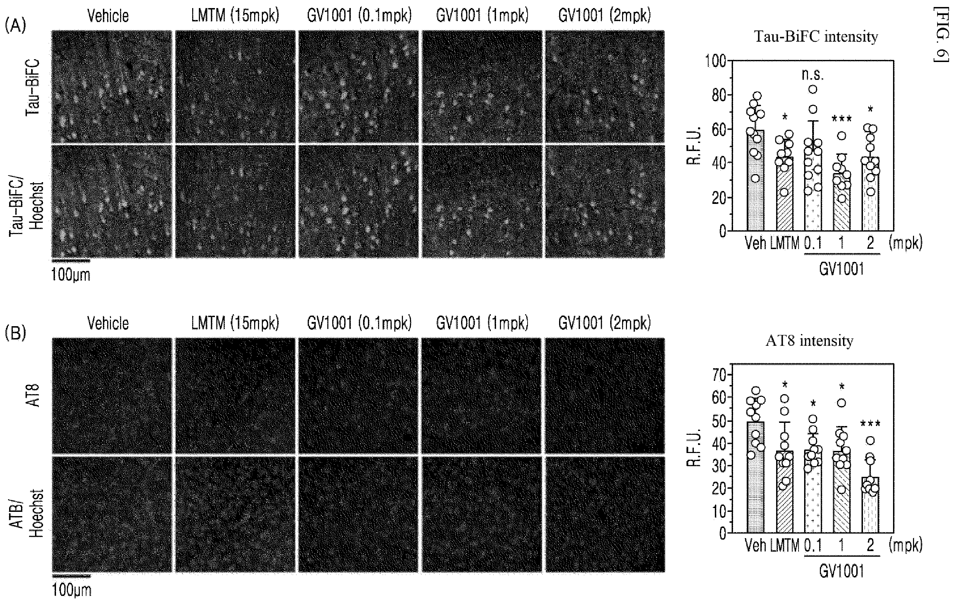

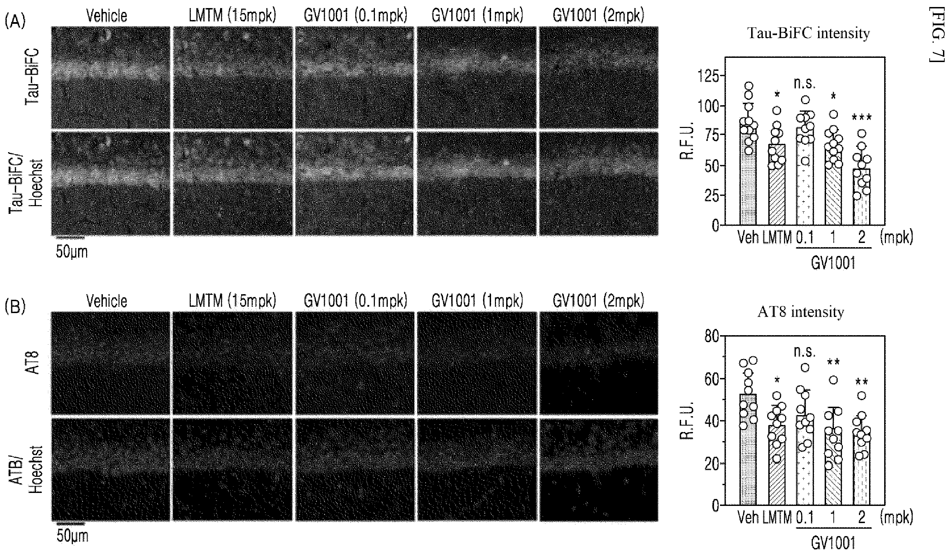

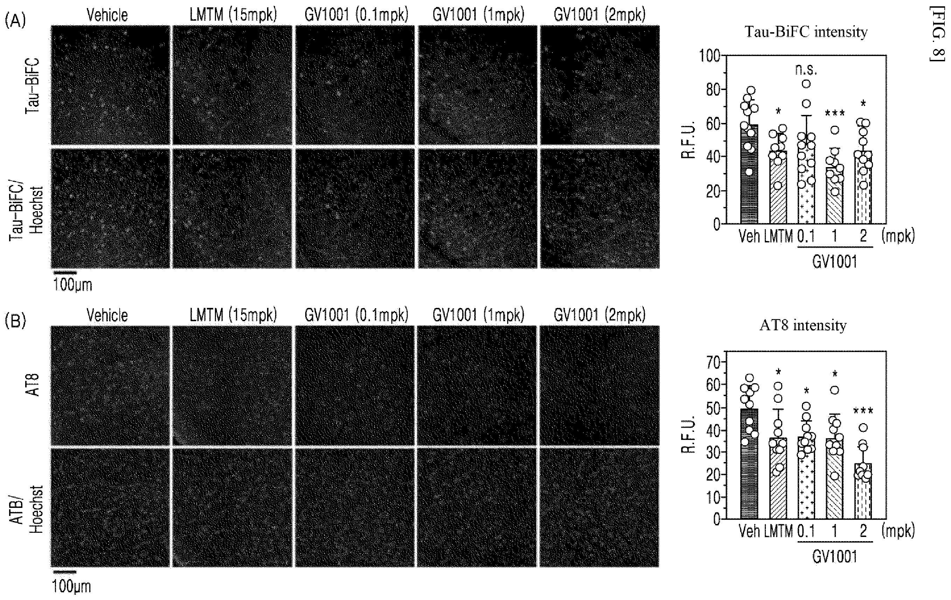

In regard to the somatosensory cortex, motor cortex, hippocampus and substantia nigra regions, photographs showing (A) Tau-BiFC imaging and (B) AT8 immunofluorescent staining results, and graph results illustrating the quantitative analysis results of the fluorescent levels were represented by: FIG. 5 (somatosensory cortex); FIG. 6 (motor cortex); FIG. 7 (hippocampus); and FIG 8 (substantia nigra region). Further, the corresponding results are listed in Table 8 below. Through monodirectional ANOVA using Dunnett's multiple-comparisons test, the above results were analyzed (*p<0.05, **p<0.01, ***p<0.001, n.s.; Insignificant for vehicle).

**[TABLE 8]**

| Immunofluorescent staining of brain tissues | | | | | | | | |
|---|---|---|---|---|---|---|---|---|
| Analysis factor | | LTMT | GV1001 | | | | | |
| | | vs. vehicle | vs. vehicle | | | vs. LMTM | | |
| | | 15 (mg/kg) | 0.1 (mg/kg) | 1 (mg/kg) | 2 (mg/kg) | 0.1 (mg/kg) | 1 (mg/kg) | 2 (mg/kg) |
| Somatosen sory cortex | TauBiFC | (15 %) | (29 %)** | (32 %)** | (37 %)*** | (16 %) | (20 %) | (25 %) |
| | AT8 | (31 %) | (25 %)* | (37 %)*** | (39 %)*** | 11% | (7 %) | (9 %) |
| Motor cortex | TauBiFC | (27 %)* | (22 %)* | (44 %)*** | (27 %)* | 6 % | (22 %) | (1 %) |
| | AT8 | (26 %)* | (26 %)* | (26 %)* | (50 %)*** | 1 % | 0 % | (32 %) |
| Hippocam pus | TauBiFC | (21 %)* | (6 %) | (23 %)* | (46 %)*** | 20 % | (1 %) | (30 %) |
| | AT8 | (28 %)* | (19 %) | (36 %)** | (34 %)** | 13 % | (10 %) | (9 %) |
| Substantia nigra | TauBiFC | (26 %) | (44 %)* | (15 %) | (32 %)* | (24 %) | 15 % | (8 %) |
| | AT8 | (16 %) | (30 %) | (32 %) | (58 %)* | (17 %) | (19 %) | (27 %) |

### Results of brain tissue imaging analysis in 4R TauP301L-BiFC mouse model

- Brain tissues of each mouse in the TauP301L-BiFC mouse model including 7-to 12-month-old mice administered with the drug 63 times for 5 months were obtained, and auto-fluorescence of the brain tissues was removed through lipid staining (Sudan black B stain), followed by conducting Tau-BiFC fluorescent imaging.
- As a result of quantitative analysis of Tau-BiFC fluorescent levels in the somatosensory cortex, motor cortex and hippocampus (CA1) regions predominantly exhibiting 4R-tauopathy due to the formation of tau oligomer inside the brain of the existing 12 month-old TauP301l-BiFC mouse model, GV1001 drug group showed significantly improved efficacy of inhibiting tau oligomer formation, as compared to the vehicle group.
- Further, as a result of evaluating exercise performance in Experimental Example 2, GV1001 drug group exhibited significant improvement in exercise performance, compared to the vehicle group. Therefore, additionally, Tau-BiFC fluorescent level of the substantia nigra region as a site which is located in the basal ganglia of the brain and has an important role in body action control was investigated. As a result, it was confirmed that Tau-BiFC fluorescent level in GV1001 0.1 mpk (mg/kg) and 2 mpk drug groups was significantly reduced as compared to the vehicle group. That is, tau oligomer formation inhibitory efficacy was confirmed.
- In the case of reference compound LMTM administration group (15 mpk), Tau-BiFC fluorescent level was significantly reduced in the motor cortex, hippocampus and substantia nigra regions, as compared to the vehicle group. That is, tau oligomer formation inhibitory efficacy was confirmed.

More specifically, in the somatosensory cortex region, it was confirmed that GV1001 0.1 mpk, 1 mpk and 2 mpk drug groups show significant reduction in Tau-BiFC fluorescent levels of 0.71 times, 0.68 times and 0.63 times, respectively, as compared to the vehicle group.

In the motor cortex region, it was confirmed that GV1001 1 mpk and 2 mpk drug groups show significant reduction in Tau-BiFC fluorescent levels of 0.56 times and 0.73 times, respectively, as compared to the vehicle group.

In the hippocampus (CA1) region, it was confirmed that GV1001 1 mpk and 2 mpk drug groups show significant reduction in Tau-BiFC fluorescent levels of 0.77 times and 0.54 times, respectively, as compared to the vehicle group.

In the substantia nigra region, it was confirmed that GV1001 0.1 mpk and 2 mpk drug groups show significant reduction in Tau-BiFC fluorescent levels of 0.56 times and 0.68 times, respectively, as compared to the vehicle group.

### Results of brain tissue imaging analysis in 4R TauP301L-BiFC mouse model (AT8 immunofluorescent staining)

- Brain tissues of each mouse in the TauP301L-BiFC mouse model including 7-to 12-month-old mice administered with the drug 63 times for 5 months were obtained. Then, after performing immunofluorescent staining using AT8 (phospho-Tau S202/T205) as a tau hyperphosphorylated antibody, the somatosensory cortex, motor cortex, hippocampus (CA1) and substantia nigra regions were subjected to quantitative analysis of AT8 fluorescent level through brain tissue fluorescent imaging. As a result, GV1001 drug group showed significant efficacy of inhibiting tau hyperphosphorylation, as compared to the vehicle group.
- In the case of reference compound LMTM administration group (15 mpk), AT8 fluorescent level was significantly reduced in the somatosensory cortex, motor cortex and hippocampus regions, as compared to the vehicle group. That is, tau hyperphosphorylation inhibitory efficacy was confirmed.

More specifically, in the somatosensory cortex region, it was confirmed that, as compared to the vehicle group, GV1001 0.1 mpk, 1 mpk and 2 mpk drug groups show significant reduction in AT8 fluorescent levels of 0.75 times, 0.63 times and 0.61 times, respectively.

In the motor cortex region, it was confirmed that GV1001 0.1 mpk, 1 mpk and 2 mpk drug groups show significant reduction in AT8 fluorescent levels of 0.74 times, 0.74 times and 0.50 times, respectively, as compared to the vehicle group.

In the hippocampus (CA1) region, it was confirmed that GV1001 1 mpk and 2 mpk drug groups show significant reduction in AT8 fluorescent levels of 0.64 times and 0.66 times, respectively, as compared to the vehicle group.

In the substantia nigra region, it was confirmed that GV1001 2 mpk drug group shows significant reduction in AT8 fluorescent level of 0.42 times, as compared to the vehicle group.

### B. Results of TauP301L-BiFC mouse-based brain lysate analysis

After obtaining the brain lysate of each mouse in the TauP301L-BiFC mouse model including 7- to 12-month-old mice administered with the drug for 5 months, immunoblot was conducted using total tau (Tau5) and phosphorylated tau (pS199, pS396) antibodies of 1) a soluble fraction sample and 2) an insoluble fraction sample and the results thereof are shown in FIG. 9 (FIG. 9A: somatosensory cortex and motor cortex; FIG. 9B: hippocampus and substantia nigra) and FIG. 10. In addition, the results of quantitative analysis of the total tau and phosphorylated tau levels were shown in graphs. Further, the above results are listed in Table 9 below. Through monodirectional ANOVA using Dunnett's multiple-comparisons test, the above results were analyzed (*p<0.05, **p<0.01, ***p<0.001, n.s.; Insignificant for vehicle).

**[TABLE 9]**

| Tau immunoblots | | | | | | | | | |
|---|---|---|---|---|---|---|---|---|---|
| Analysis factor | | | LMTM | GV1001 | | | | | |
| | | | vs. vehicle | vs. vehicle | | | vs. LMTM | | |
| | | | 15 (mg/kg) | 0.1 (mg/kg) | 1 (mg/kg) | 2 (mg/kg) | 0.1 (mg/kg) | 1 (mg/kg) | 2 (mg/kg) |
| Soluble fraction | Total tau | hTau | (20%) | (18%) | (19%) | (21%)* | 4% | 2% | 0% |
| | | mTau | (24%)** | (23%)** | (31%)** * | (29%)** * | 1% | (9%) | (7%) |
| | Phosp horyl ated tau | hTau (pS199) | (20%) | (26%)* | (23%) | (43%)** * | (8%) | (4%) | (30%) |
| | | mTau (pS199) | (34%)** * | (42%)** * | (44%)** * | (54%)** * | (13%) | (15%) | (31%) |
| | | hTau (pS396) | (23%) | (33%)* | (32%)* | (49%)** * | (12%) | (12%) | (33%) |
| | | mTau (pS396) | (27%)* | (38%)** * | (38%)** * | (50%)** * | (15%) | (15%) | (32%) |
| Insoubl e fraction | Total tau | | (42%) | (81%)** | (66%)* | (71%)* | (68%) | (42%) | (50%) |

| | | | | | | | | | |
|---|---|---|---|---|---|---|---|---|---|
| (*p<0.05, **p<0.01, ***p<0.001) | | | | | | | | | |

### Results of tau immunoblot assay utilizing brain lysate in 4R TauP301L-BiFC mouse model

- Brain lysate-soluble fraction and insoluble fraction of the TauP301L-BiFC mouse model including 7- to 12-month-old mice administered with the drug 63 times for 5 months were secured. Then, utilizing the above fractions, tau immunoblot was conducted using total tau (Tau5) and phosphorylated tau (pS199, pS396) antibodies. Then, the total tau and phosphorylated tau were subjected to quantitative analysis of band intensities.
- When conducting the brain lysate tau immunoblot of the TauP301L-BiFC mouse model, human Tau-BiFC (hTau-VN173, hTau-VC155) exhibited bands in sizes of 85 kDa and 76 kDa, while the mouse Tau (mTau) exhibited bands in a size of 50 to 70 kDa.
- As a result of tau immunoblot utilizing the soluble fraction, GV1001 drug group showed significant reduction in total tau and phosphorylated tau levels, as compared to the vehicle group. That is, efficacy of inhibiting the soluble tau oligomer and phosphorylated tau formation was confirmed.
- Further, even in the result of tau immunoblot utilizing the insoluble fraction, GV1001 drug group showed significant reduction in total tau level, as compared to the vehicle group. That is, it was confirmed that GV1001 drug exhibits insoluble tau aggregate formation inhibitory efficacy.
- In the case of reference compound LMTM administration group (15 mpk), total tau and phosphorylated tau levels were significantly reduced in each mouse, as compared to the vehicle group.

More specifically, the soluble fraction was as follows.
- In the case of total tau, total hTau was significantly reduced by 0.79 times in GV1001 2 mpk drug group, as compared to the vehicle group. Further, GV1001 0.1 mpk, 1 mpk and 2 mpk drug groups showed significant reduction in total mTau of 0.77 times, 0.69 times and 0.71 times, respectively.
- In the case of phosphorylated tau (pS199), hTau pS199 was significantly reduced by 0.74 times and 0.57 times in GV1001 0.1 mpk and 2 mpk drug groups, respectively, as compared to the vehicle group. Further, GV1001 0.1 mpk, 1 mpk and 2 mpk drug groups showed significant reduction in mTau pS199 of 0.58 times, 0.56 times and 0.46 times, respectively.
- In the case of phosphorylated tau (pS396), hTau pS396 was significantly reduced by 0.67 times, 0.68 times and 0.51 times in GV1001 0.1 mpk, 1 mpk and 2 mpk drug groups, respectively, as compared to the vehicle group. Further, GV1001 0.1 mpk, 1 mpk and 2 mpk drug groups showed significant reduction in mTau pS396 of 0.62 times, 0.62 times and 0.50 times, respectively.

The insoluble fraction was as follows.
- The total tau level was significantly reduced by 0.19 times, 0.34 times and 0.29 times in GV1001 0.1 mpk, 1 mpk and 2 mpk drug groups, respectively, as compared to the vehicle group.

The terms used herein are intended only for the purpose of describing specific embodiments and are not intended to limit the invention. Terms in which number is omitted before a noun are not intended to limit quantity, but indicate that there is more than one of the noun items mentioned.

Recitation of ranges of numeral values is merely because it is a shorthand method of referring individually to each separate value falling within that range. Further, unless otherwise indicated, it is incorporated herein like as if each separate value was individually recited in the specification. The end value of every range is included within that range and can be independently combined.

All methods recited herein can be performed in any order unless otherwise indicated or otherwise clearly contradicted by context. The use of any and all embodiments or exemplary language (e.g., "such as"), unless included in the claims, is merely to better describe the invention, but it is not intended to limit the scope of the present invention. No language in the specification should be construed as indicating any non-claimed element as essential to the practice of the invention. Unless otherwise defined, technical and scientific terms used herein have the same meaning as commonly understood by a person of ordinary skill in the art to which this invention pertains.

Preferred embodiments of the present invention include the most optimal mode known to the inventor for carrying out the present invention. Variations of preferred embodiments may become apparent to those skilled in the art upon reading the foregoing description. The inventors expect that those skilled in the art can employ such variations as appropriate, and also expect that the invention is to be practiced otherwise than as described herein. Accordingly, this invention includes all modifications and equivalents of the subject matter recited in the appended claims, as permitted by patent law. Moreover, any combination of the above-mentioned elements in all possible variations is encompassed by the present invention unless explicitly stated herein to the contrary or clearly contradicted by context. Although the present invention has been specifically shown and described with reference to exemplary embodiments, those skilled in the art will appreciate that various changes may be made in the form and details without departing from the spirit and scope of the invention as defined by the following claims.

## Claims

1. A pharmaceutical composition for preventing or treating 4R tauopathy, comprising, as an active ingredient, one or more peptides selected from the group consisting of a peptide having an amino acid sequence of SEQ ID NO: 1, a peptide having at least 80% sequence identity to the amino acid sequence of SEQ ID NO: 1, and a fragment thereof.

2. The pharmaceutical composition according to claim 1, wherein the fragment includes a peptide consisting of three or more amino acids.

3. The pharmaceutical composition according to claim 1 or 2, wherein the 4R tauopathy is selected from progressive supranuclear palsy, corticobasal degeneration, argyrophilic grain disease, globular glial tauopathy and ARTAG (aging-related tau astrogliopathy including globular glial tauopathy).

4. A food composition for preventing or improving 4R tauopathy, comprising, as an active ingredient, one or more peptides selected from a peptide having an amino acid sequence of SEQ ID NO: 1, a peptide having at least 80% sequence identity to the amino acid sequence of SEQ ID NO: 1, and a fragment thereof.

5. The food composition according to claim 4, wherein the fragment includes a peptide consisting of three or more amino acids.

6. The food composition according to claim 4 or 5, wherein the 4R tauopathy is selected from progressive supranuclear palsy, corticobasal degeneration, argyrophilic grain disease, globular glial tauopathy and ARTAG (aging-related tau astrogliopathy including globular glial tauopathy).
